# EUROPÄISCHE PATENTANMELDUNG

(11) **EP 1 277 444 A2**
(43) Veröffentlichungstag der Anmeldung: **22.01.2003**
(21) Anmeldenummer: 02010509.4
(22) Anmeldetag: 10.05.2002
(51) Int. Cl.: A61B 17/70

(54) **Gewindebolzen, Gewindemutterabschnitt und Gewindeverbindung**

(30) Priorität: 20.07.2001 CH 13552001
(71) Anmelder: Hermann, Werner, 6312 Steinhausen (CH)
(72) Erfinder: Hermann, Werner, 6312 Steinhausen (CH)
(74) Vertreter: Heusch, Christian

(57) **Zusammenfassung**

Gewindeverbindung, mit einem Gewindebolzen (**10**), der ein Bolzengewinde (**12**) aufweist, und mit einem Gewindemutterabschnitt (**21**), der ein Muttergewinde (**22**) aufweist. Mit dem Gewindebolzen wird beispielsweise ein Fixateurelement (**30**) in einer Elementaufnahme (**24**) einer Pedikelschraube (**20**) fixiert. Das Bolzengewinde (**12**) weist eine Achse (**A**), eine beim Einschrauben vorlaufende Flanke **(FB**_{**α**}**)** und eine beim Einschrauben nachlaufende Flanke **(FB**_{**ω**}**)** auf. Das Muttergewinde **(22)** ist im Wesentlichen komplementär zum Bolzengewinde (**12**) ausgebildet. Am Bolzengewinde (**12**) weist die nachlaufende Flanke (**FB**_{ω}) gegenüber einer zur Achse (**A**) normalen Ebene (**E**) mindestens in einem Flankenabschnitt einen Flankenwinkel (ω) auf, der gleich oder grösser ist als 0°.

## Beschreibung

Die Erfindung betrifft einen Gewindebolzen nach dem Oberbegriff des Anspruchs **1**, einen Gewindemutterabschnitt nach dem Oberbegriff des Anspruchs **7** und eine Gewindeverbindung nach dem Oberbegriff des Anspruchs **13**.

Gewindeverbindungen dieser Art dienen unter Anderem zur Fixierung eines Fixateurelementes in Implantatelementen, die ihrerseits, meist unmittelbar, in Knochenmaterial implantiert sind. Eine Implantatanordnung, die zur Korrektur einer deformierten Wirbelsäule benutzt wird, weist als Fixateurelement im Allgemeinen eine in geeigneter Weise leicht gebogene Stange auf, welche im wesentlichen den korrigierten Verlauf einer Wirbelsäule definiert. Bei den Implantatelementen, die direkt im Knochenmaterial implantierbar sind, kann es sich um Schanzschrauben oder um Pedikelschrauben handeln, die mit ihrem gewindeten Ende unmittelbar in Knochenbereichen bzw. in Wirbelfortsätzen eines Patienten implantierbar sind. Für ein stangenartiges Fixateurelement werden üblicherweise mehrere, mindestens aber zwei, Schanz- oder Pedikelschrauben benötigt.

Schanzschrauben sind eigentliche Gewindestifte mit langen Schäften, die implantiert werden. Dank ihrer langen Schäfte eignen sie sich auch ohne Benutzung einer zusätzlichen Zange zum Reponieren verschobener Wirbelteile. Der Nachteil der Schanzschrauben liegt darin, dass die langen Schäfte nach der Implantierung mit Hilfe einer Scherzange auf die erforderliche Länge gekürzt werden müssen, und dass nach dem Kürzen stets ein scharfkantiger rückwärtiger Bereich von wenigen mm Länge verbleibt.

Pedikelschrauben weisen keinen verlängerten Schaft auf, so dass sie zum Reponieren von Wirbelteilen mit Hilfe einer Reponierzange angefasst werden müssen. Dafür entfällt das nachträgliche Abscheren des verlängerten Schaftes. Den Pedikelschrauben wird auch ein weiterer Vorteil, nämlich derjenige der relativ hohen Belastbarkeit auf Zug, zugeschrieben.

Zur Fixierung des stangenartigen Fixateurelementes an den Schanz- oder Pedikelschrauben sind verschiedene Anordnungen bekannt. Beispielsweise können Klemmkörper verwendet werden, wobei jeder Klemmkörper einen Durchbruch für das Fixateurelement aufweist, in welchem das Fixateurelement mittels einer Klemmschraube befestigt wird. Der Klemmkörper seinerseits ist an der Schanzoder Pedikelschraube angeordnet. Das gesamte Implantat besteht hierbei aus den folgenden Bestandteilen: (1) dem stangenartigen Fixateurelement; (2) den Klemmkörpern; (3) den Klemmschrauben; und (4) den Schanz- oder Pedikelschrauben. Mit dieser Anordnung erreicht man eine einwandfreie, haltbare und gut montierbare Fixierung des stangenartigen Fixateurteiles in den im Knochenmaterial implantierten Implantatelementen. Als Implantatelemente können, wie erwähnt, Schanz- oder Pedikelschrauben verwendet werden, wobei ggfs. für ein Fixateurelement sowohl Schanzschrauben wie auch Pedikelschrauben benutzt werden können.

Einige Operateure ziehen Implantate mit einer geringeren Anzahl von Bestandteilen vor. Bekannt ist eine Anordnung, die sich allerdings nicht mit Schanzschrauben sondern nur mit Pedikelschrauben realisieren lässt, die aber mit nur drei Arten von Bestandteilen auskommt, nämlich mit (1) dem stangenartigen Fixateurelement; (2) den Fixierschrauben; und (3) den Pedikelschrauben. Die Pedikelschrauben weisen an ihrem posterioren Ende, also im Bereich des Schraubenkopfes bzw. an Stelle eines Schraubenkopfes eine geschlitzte Ausnehmung mit Muttergewinde auf, die eine Elementaufnahme für das stangenförmige Fixateurelement bildet; Pedikelschrauben dieser Art werden auch als Tulpenschrauben bezeichnet, da ihr posteriores Ende eine leichte Ähnlichkeit mit einer Tulpenblüte aufweist. Das Fixateurelement wird mit Hilfe der Fixierschrauben in den Elementaufnahmen der Pedikelschrauben befestigt. Die Fixierschrauben sind so bemessen, dass sie mit ihrem Frontende das Fixateurelement auf den Grund der rinnenartigen Ausnehmung vorspannen. Zwar ist bei dieser Anordnung die Anzahl der verwendeten Bauteile gering, aber es lässt sich keine befriedigende Fixierung des stangenartigen Fixateurelementes an den Pedikelschrauben erzielen. Ein wesentlicher Nachteil der Anordnung liegt darin, dass die Fixierschraube, welche das stangenartige Fixateurelement in der rinnenartigen posterioren Ausnehmung der Pedikelschraube halten soll, unter Belastung die Tendenz hat, die beiden Seitenteile, welche die rinnenartige Ausnehmung begrenzen, so zu deformieren, dass diese aufgespreizt werden.

Dies ist insbesondere eine Folge der Ausbildung des an der Elementaufnahme und der Fixierschraube verwendeten Gewindes, welches herkömmlicherweise ein Sägezahngewinde ist. Das Muttergewinde ist nicht umlaufend sondern wegen der tulpenförmigen posterioren Ausbildung der Pedikelschraube nur an den zwei Wandungen vorhanden, welche die rinnenartige Ausnehmung begrenzen. Das Bolzengewinde des Sägezahngewindes weist eine Achse, eine beim Einschrauben vorlaufende Flanke und eine beim Einschrauben nachlaufende Flanke auf. Die beim Einschrauben vorlaufende Flanke ist gegenüber einer Normalebenen zur Achse um 30° nach rückwärts geneigt, das heisst, ihr Flankenwinkel beträgt bei einem DIN-Normgewinde + 30°. Die beim Einschrauben nachlaufende Flanke ist gegenüber einer Normalebene zur Achse nach vorne geneigt, das heisst, ihr Flankenwinkel ist negativ und beträgt bei einem DIN-Normgewinde - 3°. Hauptsächlich infolge dieser Vorwärts-Neigung der nachlaufenden Flanke übt ein solches Bolzengewinde unter den nach dem Implantieren unvermeidlichen Bewegungen Kräfte auf das komplementäre Muttergewinde aus, die die Tendenz haben, dieses aufzuweiten. Dies ist im vorliegenden Falle, wo das Muttergewinde nicht umlaufend sondern durch die Ausnehmung unterbrochen ist, besonders gravierend. Die Wandungen, welche die rinnenartige Ausnehmung seitlich begrenzen, werden auseinander gespreizt. Dies kann durch ein stärkeres Anziehen der Schraubenverbindung nicht wettgemacht werden, denn ein stärkeres Anziehen würde gleichzeitig die Spreizwirkung fördern. Die Folge davon ist, dass keine einwandfreie dauerhafte Fixierung des Fixateurelementes erzielt werden kann.

Es liesse sich einwenden, dass die erwähnte unerwünschte Spreizwirkung vermieden werden könnte, wenn das posteriore Ende der Pedikelschraube mit einem Bolzengewinde versehen würde und anstelle der Fixierschraube eine Fixiermutter verwendet würde. Dies würde aber die Montage wesentlich erschweren, insbesondere da eine solche Fixiermutter bedeutend geringere Abmessungen aufweisen würde als die Fixierschraube; Bestandteile mit kleinen Abmessungen werden aber bekanntlich möglichst vermieden; einerseits ist es schwierig, kleine Bestandteile und die dazugehörigen, ebenfalls kleinen Werkzeuge zu manipulieren, und anderseits ist es äusserst schwierig, allenfalls in eine Operationswunde gefallene kleine Bestandteile zu detektieren und zu entfernen.

Die Tatsache, dass die oben beschriebene Lösung mit den sogenannten Tulpenschrauben unzulänglich ist, ist schon seit Längerem bekannt, aber die Gründe für diese Unzulänglichkeit, die wie oben beschrieben mit der Art des verwendeten Gewindes im Zusammenhang stehen, wurden nicht erkannt. Dennoch wurde - allerdings erfolglos - versucht, die Lösung mit den sogenannten Tulpenschrauben zu verbessern. Dazu wurde zum Beispiel eine Anordnung entwickelt, bei welcher jede der im Knochen befestigbaren Pedikelschrauben im posterioren Bereich eine zur Seite offene Ausnehmung aufweist, welche die Elementaufnahme bildet. Nach Einführung des Fixateurelementes in die Elementaufnahmen wird über jede dieser Ausnehmungen eine Überwurfhülse geschoben, die anschliessend mit Hilfe einer Fixierschraube befestigt wird. Es werden somit die folgenden Bestandteile verwendet: (1) das stangenartige Fixateurelement; (2) die Implantatstifte bzw. Pedikelschrauben; (3) die Überwurfhülsen; und (4) die Fixierschrauben. Bei dieser Anordnung ist die Anzahl der verwendeten Bestandteile wieder gleich wie bei der weiter oben beschriebenen Lösung mit den Klemmkörpern, mit dem zusätzlichen Nachteil, dass die Überwurfhülsen und die Fixierschrauben sehr klein und daher schwierig manipulierbar sind. Das Ergebnis dieser Anordnung ist dennoch unbefriedigend.

Mit der vorliegenden Erfindung soll nun die Anordnung zur Befestigung eines Fixateurelementes in einer Pedikelschraube in der Art einer Tulpenschraube so verbessert werden, dass die Nachteile des Standes der Technik vermieden oder mindestens stark reduziert werden.

**Aufgabe** der Erfindung ist es daher, ausgehend von einer Anordnung mit einer Tulpenschraube,
- einen Gewindebolzen der eingangs genannten Art so zu modifizieren, dass der unerwünschte Effekt des Aufspreizens vermieden wird;
- einen Muttergewindeabschnitt zu schaffen, welcher sich zur Verwendung mit einem solchen Gewindebolzen eignet; und
- eine Gewindeverbindung vorzuschlagen, in welcher der Gewindebolzen nach der Erfindung mit einem Gewindemutterabschnitt nach der Erfindung kombiniert ist.

Die **Lösung** dieser Aufgabe erfolgt erfindungsgemäss
- für den Gewindebolzen durch die Merkmale des kennzeichnenden Teils des Anspruchs **1**;
- für den Gewindemutterabschnitt durch die Merkmale des kennzeichnenden Teils des Anspruchs **7**; und
- für die Gewindeverbindung durch die Merkmale des kennzeichnenden Teils des Anspruchs **13**.

Weiterbildungen der Erfindung werden durch die jeweiligen abhängigen Ansprüche definiert.

Die Ausbildung des Gewindes der neuen Gewindeverbindung unterscheidet sich von einem herkömmlichen Sägezahngewinde dadurch, dass nicht nur die beim Einschrauben vorlaufende Flanke rückwärts geneigt ist bzw. einen positiven Flankenwinkel aufweist, sondern dass die beim Einschrauben nachlaufende Flanke keine Neigung oder aber eine Rückwärts-Neigung bzw. einen Flankenwinkel von 0° oder mehr als 0° aufweist. Die Rückwärts-Neigung der nachlaufenden Flanke erstreckt sich mindestens über einen Teil der radialen Ausdehnung der Flanke. Der unmittelbare Effekt dieser neuartigen Ausbildung der Gewindeverbindung besteht darin, dass an Stelle der Spreizwirkung, die das herkömmliche Sägezahngewinde ausübt, eine Verankerungswirkung und Selbsthemmungswirkung zustande kommt. Eine tendenzielle Rückwärtsbewegung des Gewindebolzens hat nämlich zur Folge, dass die bei der Rückwartsbewegung bzw. beim Ausschrauben vorlaufenden Flanken (die beim Einschrauben die nachlaufenden Flanken sind) eine einer Spreizwirkung entgegengesetzte Wirkung auf das Muttergewinde bzw. auf die Wandungen ausüben, welche die rinnenartige Ausnehmung begrenzen, die die Elementaufnahme für das Fixateurelement bildet. Auf diese Weise kommt eine einwandfreie und haltbare Fixierung des Fixateurelementes zustande, ohne dass eine vermehrte Anzahl von Bestandteilen verwendet werden müsste.

Eine Gewindeverbindung mit einem Bolzengewinde nach der Erfindung und mit einem komplementären Muttergewinde nach der Erfindung ergibt nicht nur eine einwandfreie selbsthemmende Fixierung des Fixateurelementes in der Art einer Verankerung, sondern sie lässt sich auch ohne besondere Schwierigkeiten wieder lösen. Insbesondere sind bei der Montage der neuen Gewindeverbindung dank der auftretenden Selbsthemmung nicht allzu grosse Anzugskräfte bzw. Anzugsmomente erforderlich, und dies hat den weiteren Vorteil, dass die Gefahr einer ungewollten Verbiegung des stangenartigen Fixateurelementes beim Anziehen der Fixierschrauben nahezu ausgeschlossen ist.

Die neue Gewindeverbindung weist einen weiteren bedeutenden Vorteil auf: Dank der Möglichkeit, das stangenartige Fixateurelement mit grösserer Sicherheit und Dauerhaftigkeit in der Elementaufnahme der Pedikelschraube zu befestigen, kann in Betracht gezogen werden, das Muttergewinde der Elementaufnahme umfangsmässig zu reduzieren. Bei herkömmlichen Pedikelschrauben in der Art von Tulpenschrauben wird, wie weiter oben beschrieben, die Elementaufnahme durch ein Paar diametral angeordneter nach hinten offene Ausnehmung am posterioren Ende der Pedikelschraube gebildet. Diese zwei diametralen Ausnehmungen haben zur Folge, dass sich das Muttergewinde nicht längs 360° erstreckt sondern in den einander gegenüberliegenden Bereichen, welche den Ausnehmungen entsprechen, Gewindeunterbrüche aufweist. Bei der Festlegung der Breite der Ausnehmungen müssen verschiedene Gesichtspunkte berücksichtigt werden. Einerseits wird eine möglichst solide Schraubenverbindung zwischen der Elementaufnahme und der Fixierschraube und eine annähernd genaue Passung des Fixateurelementes in der Elementaufnahme angestrebt; dazu sollte die Ausnehmungen möglichst schmal und das Muttergewinde nur geringfügig, soweit erforderlich, unterbrochen sein. Anderseits sollte ein gewisser Spielraum für das Fixateurelement innerhalb der Elementaufnahme vorhanden sein, da sonst die Einschraubtiefe der Pedikelschraube genau vorgegeben ist und/oder das stangenartige Fixateurelement örtlich stark gebogen sein muss; dazu sollten die Ausnehmungen möglichst breit sein oder es sollte nicht nur ein Paar diametral angeordneter Ausnehmungen sondern mehrere Paare diametral angeordneter, umfangsmässig gegeneinander versetzter Ausnehmungen vorgesehen sein; beides kommt einer beträchtlichen umfangsmässigen Reduktion des Muttergewindes gleich. Bei herkömmlichen Lösungen mit Pedikelschrauben in der Art von Tulpenschrauben und mit Fixierschrauben, welche die üblichen Sägezahngewinde aufweisen, kann ohnehin, wie schon mehrfach erwähnt, keine sicherere Schraubverbindung realisiert werden; eine beträchtliche umfangsmässige Reduktion des Muttergewindes ist daher ausgeschlossen, so dass man sich mit den erwähnten Nachteilen einer fest definierten Einschraubtiefe für die Pedikelschrauben und/oder einer örtlich starken Verbiegung der Fixierstange abfinden muss. Die Verwendung von Elementaufnahmen und Fixierschrauben mit einem Gewinde nach der Erfindung erlaubt nun die Realisation einer stark verbesserten, guten, sicheren und dauerhaften Fixierung des Fixateurelementes in der Elementaufnahme der Pedikelschrauben, so dass eine beträchtliche umfangsmässige Reduktion des Muttergewindes in Kauf genommen werden kann. Durch eine solche Reduktion des Muttergewindes wird vermieden, dass eine genau definierte Einschraubtiefe der Pedikelschraube eingehalten werden muss und/oder dass das Fixateurelement stark gebogen werden muss.

Ein weiterer Vorteil der neuen Gewindeverbindung liegt darin, dass die Verwendung einer nicht passenden Fixierschraube, die entweder das Muttergewinde an der Pedikelschraube beschädigen würde oder eine nicht einwandfreie Fixierung des Fixateurelementes zur Folge haben könnte, praktisch ausgeschlossen.

Im Weiteren ist die Gefahr einer Verkantung beim Einschrauben im Vergleich mit herkömmlichen Gewinden stark reduziert; auch dadurch wird die Gefahr von Beschädigungen verringert.

Eine Rundung oder ein Anschrägen der in Richtung der Schraubenbolzenachsen verlaufenden Kanten der Ausnehmung bzw. Ausnehmungen verhindert Verletzungen und kann ebenfalls dazu beitragen, das Verkanten zu verhindern.

Wie schon erwähnt, kann sich ggfs. die Rückwärts-Neigung der beim Einschrauben nachlaufenden Flanke nur über einen Teil der radialen Ausdehnung der Flanke erstrecken; bei einer geeigneten Formgebung der Flanke kann dadurch die Verankerungswirkung sogar gesteigert werden.

Im Allgemeinen werden die neuen Bolzen- und Muttergewinde aber so ausgebildet, dass die Flanke im Längsschnitt im wesentlichen gerade begrenzt ist, was nicht ausschliessen soll, dass sie im Bereich von Kern und Kopf die üblichen Abrundungen oder Kantenbrechungen aufweisen.

Bei einem bevorzugten Bolzengewinde nach der Erfindung beträgt der Flankenwinkel der beim Einschrauben vorlaufenden Flanke genau oder mindestens annähernd + 30°, während der Flankenwinkel der beim Einschrauben nachlaufenden Flanke zwischen 0° und etwa + 30° liegen kann, wobei sich Flankenwinkel im Bereich von + 10° bewährt haben.

Ein Vorteil bei der Verwendung stark geneigter nachlaufender Flanken kann darin gesehen werden, dass sich mit einer Zunahme des Flankenwinkels die Flankenfläche vergrössert. Dies hat den günstigen Effekt, dass ein bestimmtes Anzugsmoment der Schraubenverbindung eine geringere Flächenpressung zur Folge hat. Geringe Flächenpressungen sind erwünscht, weil dadurch die Gefahr von Mikro-Beschädigungen der Oberfläche verringert wird.

Die neuen Gewindebolzen können als Stiftschrauben, zusammen mit geeigneten separaten Muttern, verwendet werden, aber es wird meist vorgezogen, sie mit einem integralen Schraubenkopf zu versehen.

Die Pedikelschraube ist im Allgemeinen so ausgebildet, dass die Längsachsen des Gewindes am Muttergewindeabschnitt einerseits und des Implantatgewindes anderseits zusammenfallen. Dies ist aber keine Notwendigkeit. Diese Achsen können parallel aber gegeneinander versetzt sein oder einen Winkel einschliessen, wenn damit eine bessere Anordnung des Fixateurelementes relativ zum das Implantelement aufnehmenden Knochenbereich erzielt werden kann.

Die Herstellung des neuen Gewindebolzens und des neuen Gewindemutterabschnitt erfolgt mit Hilfe von Spezialwerkzeugen mit jeweils komplementärer Form und vorzugsweise auf CNC-gesteuerten Automaten. Es ist nicht möglich, das Gewinde am Gewindebolzen bis zu ursprünglichem vorderen Ende zu erzeugen, da beim Zurückziehen des Werkzeuges der vorderste Gewindegang beschädigt bzw. angeschnitten wird. Um dennoch eine einwandfreie Schraubenverbindung zu erhalten, ist es vorteilhaft, wenn der ursprünglich vorderste Bereich des Gewindebolzens abgestochen wird. Dies erlaubt es auch, eine einwandfreie Anlagefläche zu erhalten, mit welcher der Gewindebolzen bzw. die Fixierschraube am Fixateurelement anliegt.

Auch bei der Herstellung des Muttergewindes in der Elementaufnahme kann das Gewinde nicht bis zum Grund der Ausnehmungen, welche die Elementaufnahme bilden, hergestellt werden. Dies ist aber ohne Konsequenzen, da die Fixierschraube wegen des Fixateurelementes ohnehin nicht bis zum Grund eingeschraubt werden muss.

Weitere Vorteile und Einzelheiten der Erfindung werden im Folgenden anhand von Ausführungsbeispielen und mit Bezug auf die Zeichnung ausführlich beschrieben. Es zeigen:
- **Fig. 1**: einen Abschnitt eines Gewindebolzens einer Fixierschraube mit dem neuen Bolzengewinde, von der Seite;
- **Fig. 2A**: eine Pedikelschraube, mit einer Elementaufnahme, in der ein stangenartiges Fixateurelement aufgenommen ist, von der Seite, teilweise geschnitten;
- **Fig. 2B**: die Elementaufnahme der in Fig. 2A dargestellten Pedikelschraube, in einer Ansicht vom posterioren Ende der Pedikelschraube;
- **Fig. 3A**: eine Variante des Bolzengewindes, in einem die Längsachse der Pedikelschraube enthaltenden Schnitt;
- **Fig. 3B**: eine weitere Variante des Bolzengewindes, in gleicher Darstellung wie Fig. 3A;
- **Fig. 3C**: eine noch weitere Variante des Bolzengewindes, in gleicher Darstellung wie die Fig. 3A und 3B;
- **Fig. 4A**: eine Variante des Gewindemutterabschnittes, in einer Ansicht vom posterioren Ende der Pedikelschraube;
und
- **Fig. 4B**: eine weitere Variante des Gewindemutterabschnittes, in gleicher Darstellung wie Fig. 4A, mit einem Abschnitt eines stangenartigen Fixateurelementes.

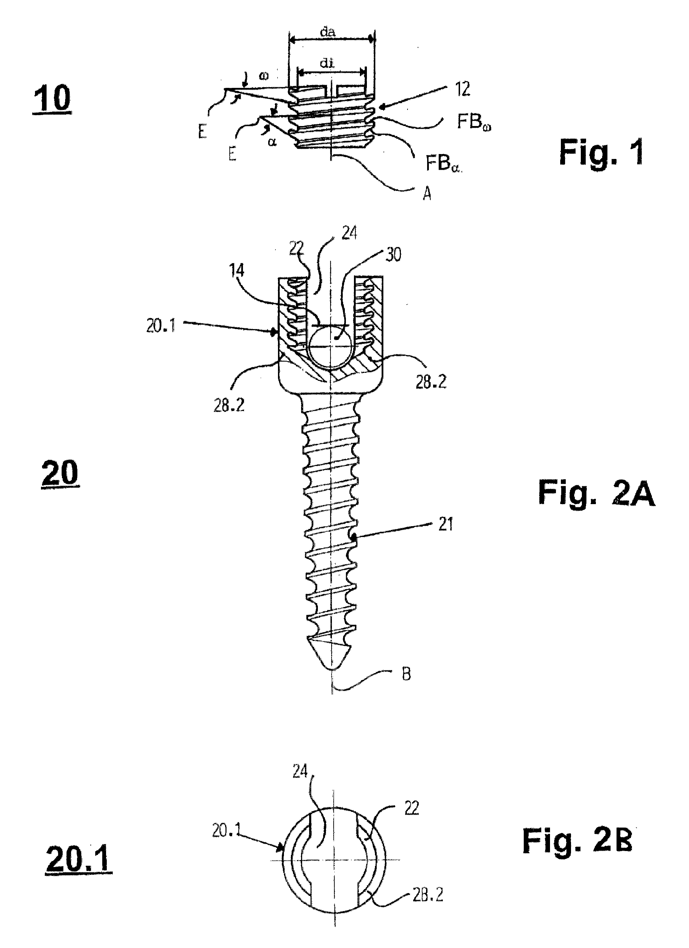

Der in **Fig. 1** dargestellte Gewindebolzen **10** einer Fixierschraube besitzt eine in Längsrichtung verlaufende Achse **A** und ein Bolzengewinde **12.** Das Bolzengewinde **12** weist einen Kerndurchmesser **d**_{**i**} und einen Kopfdurchmesser **d**_{**a**} auf. Ein Pfeil **I** zeigt die Einschraub-Richtung an. Eine Flanke **F**_{**α**}**,** die nur in einem der Gewindegänge bezeichnet ist, ist so angeordnet, dass sie beim Einschrauben des Gewindebolzens **10** vorläuft, während eine weitere Flanke **F**_{ω} so angeordnet ist, dass sie beim Einschrauben des Gewindebolzens **10** nachläuft. Eine Normalebene **N,** die in **Fig. 1** als Gerade erscheint, verläuft senkrecht zur Achse **A.** Gegenüber der Normalebene **N** ist die Flanke **F**_{**α**} rückwärts geneigt, und zwar um einen Flankenwinkel α, der im vorliegenden Ausführungsbeispiel über die radiale Ausdehnung der Flanke, mit Ausnahme von Rundungen im Kern- und Kopfbereich, konstant ist und 30° beträgt. Die Flanke **F**_{ω} ist gegenüber der Normalebene **N** ebenfalls rückwärts geneigt, und zwar um einen Flankenwinkel ω, der im vorliegenden Ausführungsbeispiel 10° beträgt. Die Flanken **F**_{α} und **F**_{ω} schliessen also einen Kopfwinkel κ von 20° ein.

Anstelle der im Schnitt geraden vorlaufenden Flanke **Fb**_{α} könnte auch eine anders ausgebildete und angeordnete vorlaufende Flanke vorgesehen sein.

**Fig. 2A** zeigt eine als Tulpenschraube ausgebildete Pedikelschraube **20** mit einer in Längsrichtung verlaufenden Achse **B.** Die Pedikelschraube **20** ist dazu bestimmt, in einen Knochen, beispielsweise einen Wirbelfortsatz, eingeschraubt zu werden. Hierzu ist ein vorderer Teil der Pedikelschraube **20** mit einem geeigneten Implantatgewinde **21** versehen. Dieses Implantatgewinde **21** kann auch anders ausgebildet sein als in **Fig. 2** dargestellt. An seinem posterioren Ende besitzt die Pedikelschraube **20** anstelle eines Schraubenkopfes einen Gewindemutterabschnitt **20.1** mit einem Muttergewinde **22**, der zu einer Elementaufnahme **24** gehört. Die Elementaufnahme **24** dient zur Aufnahme eines Fixateurelementes **30** und weist ein Paar radialer, diametral angeordneter, vom posterioren Ende ausgehender und somit nach hinten offener Ausnehmungen **26** auf, die von zwei seitlichen, nicht ganz halbkreisförmigen Wandungen **28.1**, **28.2** begrenzt sind. Die Wandungen **28.1, 28.2** weisen im Schnitt etwa die Form einer zweizinkigen Gabel auf. Die einander zugewandten Flächen der Wandungen **28.1, 28.2** sind mit dem Muttergewinde **22** versehen, welches, natürlich unter Einhaltung des notwendigen Spieles bzw. der notwendigen Toleranzen, komplementär zum Bolzengewinde **12** gemäss **Fig. 1** ausgebildet ist. Das Muttergewinde **22** erstreckt sich wegen der diametralen Ausnehmungen **26** nicht längs des gesamten Umfanges sondern weist zwei Gewindeunterbrüche auf.

In **Fig. 2A** ist im Weiteren das stangenartige Fixateurelement **30** eingezeichnet, das auf dem Grund der rinnenartigen Elementaufnahme **24**, welche durch die Ausnehmungen **26** gebildet sind, aufliegt. Sodann ist eine Frontfläche **14** des Gewindebolzens **10** sichtbar, welche am Fixateurelement **30** anliegt und dieses auf den Grund der rinnenartigen Elementaufnahme **24** vorspannt und dadurch fixiert. Auf diese Weise ist das Fixateurelement **30** unmittelbar an der Pedikelschraube **20** und mittelbar, in nicht dargestellter Weise, an einem Knochenteil befestigbar.

**Fig. 2B** zeigt den Gewindemutterabschnitt **20.1** der Pedikelschraube **20,** gesehen vom posterioren Ende der Pedikelschraube **20.**

Es sei noch darauf hingewiesen, dass das Fixateurelement **30** auch einen anderen Querschnitt als einen Kreisquerschnitt aufweisen kann.

Im montierten Zustand einer Gewindeverbindung mit einem Bolzengewinde **12** gemäss **Fig. 1** und einem Muttergewinde **22** gemäss **Fig. 2** fallen die Achse A des Gewindebolzens **10** und die Achse **B** der Pedikelschraube **20** bzw. des Gewindemutterabschnitts **21** zusammen.

Die Stellung der Flanken **F**_{ω}, die den positiven Flankenwinkel ω aufweisen und somit rückwärts geneigt bzw. entgegengesetzt zur Einschraubrichtung **I** gerichtet ist, hat zur Folge, dass die neue Gewindeverbindung selbsthemmend ist und ein Fixateurelement **30** sicher festhält. Dank der neuen Stellung bzw. Rückwärts-Neigung der Flanke **F**_{ω} entsteht gewissermassen eine Ankerwirkung und es wird verhindert, dass die Wandungen **28.1, 28.2** auseinander gespreizt werden und dadurch die Elementaufnahme **24** erweitert wird. Sowohl ein unwillkürliches Lösen der Gewindeverbindung wie auch eine blosse Lockerung des Fixateurelementes **30** werden auf diese Weise wirkungsvoll verhindert.

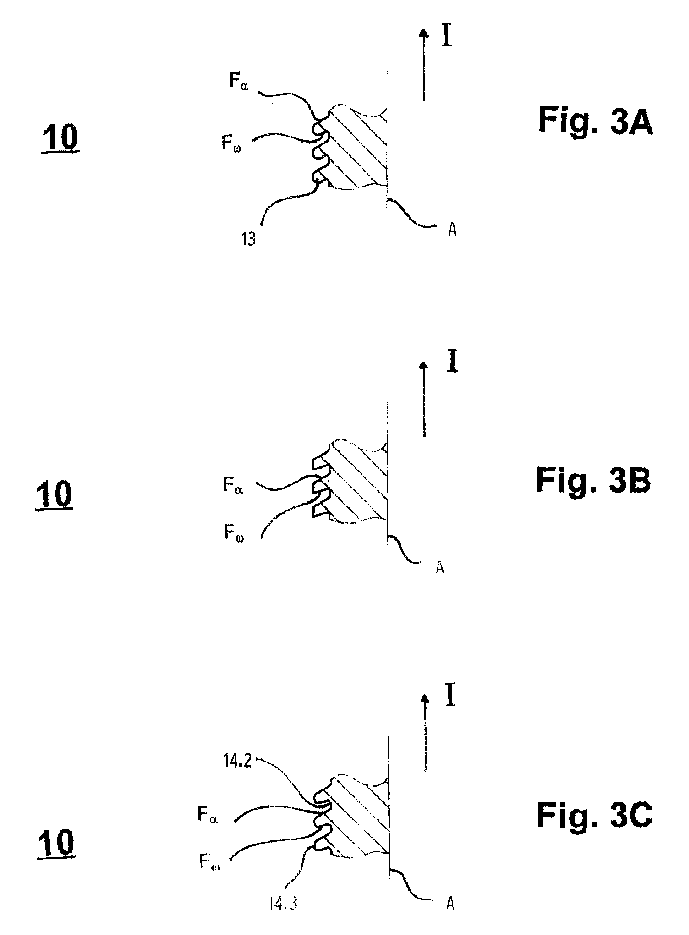

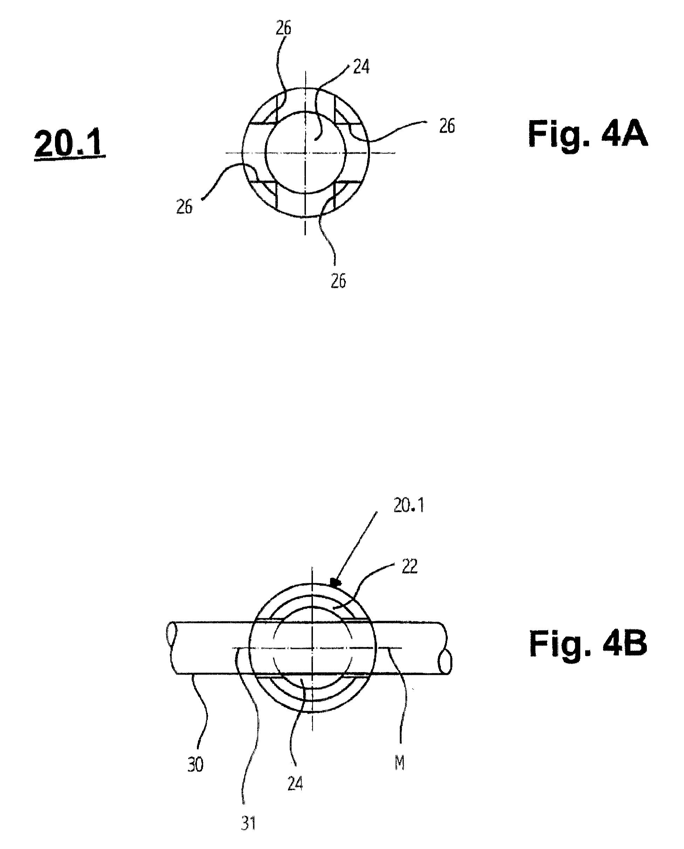

Die **Fig. 3A, 3B, 3C, 4A und 4B** zeigen, jeweils in vereinfachter Darstellung, einige der Varianten der Erfindung, die im Rahmen der vorliegenden Ansprüche realisiert werden können.

In **Fig. 3A** ist ein Teil eines Gewindebolzens **10** dargestellt, der ein Bolzengewinde **12** aufweist, dessen nachlaufende Flanke **F**_{ω} im Schnitt nicht gerade verläuft sondern einen Vorsprung **13** aufweist. Hierbei ist der Flankenwinkel ω nicht längs der ganzen radialen Ausdehnung der nachlaufenden Flanke **F**_{ω} grösser als 0° sondern nur in einem begrenzten Bereich.

**Fig. 3B** zeigt einen Teil eines Gewindebolzens **10** mit einem Bolzengewinde **12,** dessen nachlaufende Flanke **F**_{ω} parallel oder mindestens nahezu parallel zur vorlaufenden Flanke **F**_{α} ist.

**Fig. 3C** zeigt einen Teil eines Gewindebolzens **10,** bei welchem die beim Einschrauben vorlaufende Flanke **F**_{α} und die beim Einschrauben nachlaufende Flanke **F**_{ω} in einem mittleren Durchmesserbereich gerade verlaufen und im Fussbereich **14.2** und Kopfbereich **14.3** stark gerundet sind.

**Fig. 4A** zeigt eine Pedikelschraube **20** mit einem Gewindemutterabschnitt **20.1**, der zwei Paare radialer Ausnehmungen, insgesamt also **4** Ausnehmungen **26**, aufweist, die gegeneinander versetzt sind. Das Muttergewinde **22** weist daher vier Gewindeunterbrüche auf. Zwischen zwei Stellungen der Pedikelschraube **20**, die es erlauben, das stangenartige Fixateurelement ohne zu starke örtliche Biegung in der Elementaufnahme aufzunehmen, liegt hierbei nur eine 90°-Drehung der Pedikelschraube, und nicht wie beim Ausführungsbeispiel der **Fig. 2A** und **2B** eine 180°-Drehung.

**Fig. 4B** zeigt eine Pedikelschraube **20** bzw. deren Gewindemutterabschnitt **20.1,** der ein paar radialer, diametral angeordnete Ausnehmungen aufweist. Die Ausnehmungen weisen aber, im Gegensatz zu den bisher beschriebenen und dargestellten Ausführungsbeispielen, eine Breite auf, die grösser ist als der Durchmesser des Fixateurelementes **30**, so dass die Lage desselben innerhalb der Elementaufnahme **24** des Gewindemutterabschnittes **20.1** den jeweiligen Verhältnissen anpassbar ist. In **Fig. 4B** ist das Fixateurelement **30** in einer mittleren Lage dargestellt, so dass die örtliche Längsachse **31** in die Mittelebene **M** der Ausnehmungen fällt bzw. in **Fig. 4B** horizontal verläuft. Bei derselben Einschraubtiefe der Pedikelschraube 20 könnte das Fixateurelement aber auch , gesehen in **Fig. 4B,** leicht schief angeordnet sein.

## Patentansprüche

1. Gewindebolzen (**10**), welcher dazu bestimmt ist, ein Fixateurelement (**30**) in einer Elementaufnahme (**24**) einer Pedikelschraube (**20**) zu fixieren, mit einem Bolzengewinde (**12**), der eine Achse (**A**), eine beim Einschrauben vorlaufende Flanke (**FB**_{α}) und eine beim Einschrauben nachlaufende Flanke (**FB**_{ω}) aufweist,
**dadurch gekennzeichnet,**
**dass** die nachlaufende Flanke (**FB**_{ω}) gegenüber einer zur Achse (**A**) normalen Ebene (**N**) mindestens in einem Flankenabschnitt einen Flankenwinkel (ω) aufweist, der gleich oder grösser ist als 0°.

2. Gewindebolzen (**10**) nach einem der Anspruch **1**,
**dadurch gekennzeichnet,**
**dass** der Flankenwinkel (α) der vorlaufenden Flanke (**FB**_{α}) gleich oder grösser ist als der Flankenwinkel (ω) der nachlaufenden Flanke (**FB**_{ω}).

3. Gewindebolzen (**10**) nach einem der Ansprüche **1** bis **2,**
**dadurch gekennzeichnet,**
**dass** der Flankenwinkel (ω) der nachlaufenden Flanke (**FB**_{ω}) zwischen 0° und annähernd 30°, vorzugsweise im Bereich von 10°, liegt.

4. Gewindebolzen (**10**) nach Anspruch **1**,
**dadurch gekennzeichnet,**
**dass** sich der Flankenabschnitt der nachlaufenden Flanke (**FB**_{**ω**}),dessen Flankenwinkel (ω) gleich oder grösser als 0° ist, im Wesentlichen über die ganze nachlaufende Flanke (**FB**_{ω}) erstreckt.

5. Gewindebolzen (**10**) nach einem der Ansprüche **1** bis **4,**
**dadurch gekennzeichnet,**
**dass** er an seinem beim Einschrauben nachlaufenden Ende einen Schraubenkopf aufweist.

6. Gewindebolzen (**10**) nach einem der Ansprüche **1** bis **5**,
**dadurch gekennzeichnet,**
**dass** das Bolzengewinde (**12**) bis zum vorderen Ende des Gewindebolzens (**10**) reicht.

7. Gewindemutterabschnitt (**20.1**) an einer zur Aufnahme eines Fixateurelementes (**30**) bestimmten Elementaufnahme (**24**) einer Pedikelschraube (**20**), mit einem Muttergewinde (**22**) mit einer Achse (**B**), einer Aussenflanke (**FM**_{α}) und einer Innenflanke (**FM**_{ω}),
**dadurch gekennzeichnet,**
**dass** mindestens ein Abschnitt der Innenflanke (**FM**_{ω}) gegenüber einer zur Achse (**B**) normalen Ebenen (**N**) einen Flankenwinkel (ω) aufweist, der gleich oder grösser ist als 0°.

8. Gewindemutterabschnitt (**20.1**) nach Anspruch **7**,
**dadurch gekennzeichnet,**
**dass** der Flankenwinkel (α) der Aussenflanke (**FM**_{α}) gleich oder grösser ist als der Flankenwinkel (ω) der Innenflanke (**FM**_{ω}).

9. Gewindemutterabschnitt **(20.1)** nach einem der Ansprüche **7** bis **8,**
**dadurch gekennzeichnet,**
**dass** der Flankenwinkel (w) der Innenflanke (**FM**_{ω}) zwischen 0° und annähernd 30°, vorzugsweise im Bereich von 10°, liegt.

10. Gewindemutterabschnitt (**20.1**) nach Anspruch **7**,
**dadurch gekennzeichnet,**
**dass** sich der Flankenabschnitt der Innenflanke (**FM**_{ω}), dessen Flankenwinkel (ω) gleich oder grösser als 0° ist, im Wesentlichen über die ganze Innenflanke (**FM**_{ω}) erstreckt.

11. Gewindemutterabschnitt (**20.1**) nach einem der Ansprüche **7** bis **10,**
**dadurch gekennzeichnet,**
**dass** die Elementaufnahme (**24**) für das Fixateurelement (**30**) durch mindestens ein Paar diametral am posterioren Ende des Gewindemutterabschnitts (**20.1**) angeordneter, nach hinten offener Ausnehmungen gebildet ist, wobei durch jede Ausnehmung am Muttergewinde (**22**) ein Gewindeunterbruch verursacht ist.

12. Gewindemutterabschnitt (**20.1**) nach einem der Ansprüche **7** bis **11**,
**dadurch gekennzeichnet,**
**dass** mindestens ein Paar Ausnehmungen (**26**) eine Breite aufweist, die grösser ist als die entsprechende Breite des Fixateurelementes (**30**).

13. Gewindeverbindung, mit welcher ein Fixateurelement (**30**) in einer Elementaufnahme (**24**) einer Pedikelschraube (**20**) fixierbar ist,
- mit einem Bolzengewinde (**12**), das an einem Gewindebolzen (**10**) einer Fixierschraube (**10**) angeordnet ist, und
- mit einem Muttergewinde (**22**), das an einem Gewindemutterabschnitt (**21**) der Pedikelschraube (**20**) angeordnet ist,
- wobei das Bolzengewinde (**12**) eine Achse (**A**), eine beim Einschrauben vorlaufende Flanke (**FB**_{α}) und eine beim Einschrauben nachlaufende Flanke aufweist (**FB**_{ω}), und
- wobei das Muttergewinde (**22**) im Wesentlichen komplementär zum Bolzengewinde (**12**) ausgebildet ist,
**dadurch gekennzeichnet,**
**dass** am Bolzengewinde (**12**) die nachlaufende Flanke (**FB**_{ω}) gegenüber einer zur Achse (**A**) normalen Ebene (**E**) mindestens in einem Flankenabschnitt einen Flankenwinkel (ω) aufweist, der gleich oder grösser ist als 0°.

14. Gewindeverbindung nach Anspruch 13,
**dadurch gekennzeichnet,**
**dass** der Flankenwinkel (α) der vorlaufenden Flanke (**FB**_{α}) des Bolzengewindes (**12**) gleich oder grösser ist als der Flankenwinkel (ω) der nachlaufenden Flanke (**FB**_{ω}).

15. Gewindeverbindung nach einem der Ansprüche **13** bis **14,**
**dadurch gekennzeichnet,**
**dass** der Flankenwinkel (ω) der nachlaufenden Flanke (**FB**_{ω}) des Bolzengewindes (**12**) zwischen 0° und annähernd 30°, vorzugsweise im Bereich von 10°, liegt.

16. Gewindeverbindung nach Anspruch **13**,
**dadurch gekennzeichnet,**
**dass** sich der Flankenabschnitt der nachlaufenden Flanke (**FB**_{ω}) des Bolzengewindes (**12**), dessen Flankenwinkel (ω) gleich oder grösser als 0° ist, im Wesentlichen über die ganze nachlaufende Flanke (**FB**_{ω}) erstreckt.
